# EUROPEAN PATENT SPECIFICATION

(11) **EP 2 879 661 B1**
(45) Date of publication and mention of the grant of the patent: **26.01.2022**
(21) Application number: 13742226.7
(22) Date of filing: 29.07.2013
(51) Int. Cl.: A61K 9/00, A61P 35/00, A61K 31/4196, A61K 9/10, A61K 47/34, A61K 47/20

(54) **INJECTABLE COMPOSITIONS COMPRISING LETROZOLE OR ANASTROZOLE**
INJIZIERBARE ZUSAMMENSETZUNGEN MIT LETROZOL ODER ANASTROZOL
COMPOSITIONS INJECTABLES COMPRENANT DU LÉTROZOLE OU DE L'ANASTROZOLE

(30) Priority: 03.08.2012 ES 201231271
(43) Date of publication of application: 10.06.2015
(73) Proprietor: Laboratorios Farmacéuticos Rovi, S.A., 28037 Madrid (ES)
(72) Inventor: FRANCO RODRÍGUEZ, Guillermo, E-28037 Madrid (ES); GUTIERRO ADURIZ, Ibon, E-28037 Madrid (ES)
(74) Representative: Elzaburu S.L.P.
(86) International application number: PCT/EP2013/065877
(87) International publication number: WO 2014/019972

(56) References cited:
- EP-A1- 2 394 663
- WO-A2-2009/060473

## Description

### FIELD AND OBJECT OF THE INVENTION

The present patent application is directed toward compositions useful in cancer therapies.

In particular, the present invention refers to a composition suitable for forming an intramuscular implant comprising a biodegradable thermoplastic polymer of polylactic acid (PLA), dimethyl sulphoxide (DMSO) and an aromatase inhibitor compound, a suitable kit for the *in situ* preparation of the composition and its use as a medicine for the treatment of breast cancer.

### BACKGROUND

Without doubt, cancer treatments need to be developed, not only new molecular entities but also pharmacological products for improving patients' quality of life. In this sense, the development of prolonged release formulation signify an advance because they enable reducing the total dose administered, increasing the duration of each dose and the number of administrations and thereby create a positive impact on the emotional state of the patient.

In this sense, in the present invention, the active ingredients letrozole and anastrozole have been selected as candidate pharmaceutical drugs for this type of prolonged release formulation because they are the first line active ingredients in the adjuvant treatment of postmenopausal women with hormone receptor-positive advanced breast cancer for whom there is no alternative therapy beyond daily administration of a tablet.

Letrozole (4,4'-(1,2,4-triazol-1-ylmethyl)dibenzonitrile) and anastrozole (2,2'-[5-(1H-1,2,4-triazol-1-ylmethyl)-1,3-phenylene]bis(2-methylpropanenitrile)) belong to a class of drugs called non-steroidal inhibitors of aromatase and their mechanism of action consists of reducing the amount of oestrogen in the body. This effect can decelerate or stop the growth of many types of cancer-producing cells in the breast that need oestrogen to grow.

Currently there is no formulation of letrozole on the market with the ability to control the release of the drug over a long period of time. The pharmaceutical drug letrozole is currently only available in tablet form for daily oral administration. The formulations of letrozole described here enable obtaining therapeutic levels of the drug in blood from the start and continuously over a period of three months, avoiding the need for daily dosing regimes and thereby improving the patient's quality of life.

In the treatment of breast cancer, as in the treatment of cancer in general, the psychological state of the patient is very important; therefore the development of a three-monthly formulation of letrozole and/or anastrozole means a substantial improvement in their quality of life, reducing the impact that would result from daily treatment. In turn, medical examinations that are carried out during monitoring of the disease are normally conducted at 3 and 6 months over the first few years, so the administration of the formulation could coincide with consultancy visits to the doctor.

Similar reasoning has led to the appearance on the market of formulations such as Zoladex^{®}, a preformed implant of goserelin for subcutaneous three-monthly application for the treatment of prostate carcinoma, and Implanon^{®}, a preformed implant of etonogestrel used as a contraceptive. However, these preformed implants show a series of disadvantages including:
- The preparation of the implants by extrusion requires the use of high temperatures, which can cause the degradation of the active ingredient and the generation of potentially toxic impurities;
- Low homogeneity of the product obtained when including active ingredients at low doses;
- Need for surgical procedures for implanting or injection of the implant using large diameter needles.

It is also possible to find in the literature some publications on implantable compositions of letrozole and/or anastrozole such as the following:
For example, WO 2008/041245 describes implantable compositions comprising a wide variety of active ingredients such as some aromatase inhibitors, including anastrozole, in a wide variety of administration forms from preformed microparticles suspended in an aqueous vehicle to formulations that gellify *in situ.* Although it is doubtful that this document can sufficiently support all the combinations of active ingredients and administration forms that may arise, the examples always refer to preformed microparticles, that is it never describes systems of forming implants directly "*in situ*"*.* Finally, it should be pointed out that none of the examples show a duration of over 60 days.

WO 2010/065358 A1 describes compositions for the administration of medicines containing testosterone and an aromatase inhibitor for continuous administration of testosterone and for preventing its conversion to estradiol. Although the description considers the possibility that the form of administration may be an implant, the only example of a form of administration is pellets.

Also, WO 2012/074883 A1 describes biodegradable compositions for administration of pharmaceutical drugs. These compositions require the use of water-insoluble solvents such as benzyl benzoate or benzyl alcohol in order to maintain the implant in a liquid or semi-solid state. These solvents have been previously shown to provide sudden releases and therefore are not suitable for the prolonged release compositions of the present invention.

Finally, US 2008/0206303 A1 describes prolonged release formulations of anastrozole comprising a PLA or PLGA polymer that can be accompanied by a wide variety of solvents; however, in the embodiments of the invention, the solvents used are benzyl alcohol and n-methyl-2-pyrrolidone (NMP), solvents that give rise to a very large burst followed by a subsequent almost zero release. In fact, the burst that was acceptable for the inventors in this document was 25-30% in one day, a very high value, and because of this none of their examples lasted more than 60 days; in particular in dogs, animals similar to humans, release did not continue longer than 35 days. Finally, no mention was made in this document of letrozole particle size nor of the importance of this factor in the behaviour of the formulation.

Therefore, it would be desirable to obtain a three-monthly formulation of letrozole and/or anastrozole for first line adjuvant treatment of breast cancer in hormone receptor-positive postmenopausal women. For this reason, the technology of implants of the invention that are formed *in situ* overcomes the majority of the drawbacks presented by current formulations based on preformed implants. It offers an alternative practical and effective therapy for the patient achieving therapeutic profiles lasting for at least 60 days.

### DESCRIPTION OF THE FIGURES

The following figures are provided to help with the interpretation of the object of the present invention, but do not imply any limitation.
FIGURE 1: Figure 1 shows the plasma levels of letrozole (in ng/mL) obtained after intramuscular administration to New Zealand white rabbits of the formulations described in example 1. The values shown correspond to the mean plasma levels obtained in three animals of each group.
FIGURE 2: Figure 2 shows the plasma levels of letrozole (in ng/mL) obtained after the intramuscular administration to New Zealand white rabbits of the formulation described in example 2. The values shown correspond to the mean plasma levels obtained in three animals of each group.
FIGURE 3: Figure 3 shows the plasma levels of letrozole (in ng/mL) obtained after intramuscular administration to New Zealand white rabbits of the formulations described in example 3. The values shown correspond to the mean plasma levels obtained in three animals of each group.

### DETAILED DESCRIPTION OF THE INVENTION

For the composition that is the object of the present invention, the term initial "burst" is understood as the ratio of the area under the curve of plasma levels of the active ingredient in living animals obtained over the first 72 hours after intramuscular administration of the product to the total area under the curve (also termed "AUC") obtained after the injection of an amount of letrozole or anastrozole.

In order to obtain a prolonged release of active ingredient suitable for the object of the present invention, the area under the curve of the burst must be less than 10% compared to the total AUC, and ideally less than 5%. Similarly, obtaining an equilibrium in the prolonged release profile of the aromatase inhibitor over at least 60 days requires that no more than 50% of the area under the curve of plasma levels is obtained over the first 30 days after the injection. That is, for the object of the present invention, the preferred prolonged release of active ingredient is such that the area under the curve of the burst is less than 10% of the total AUC and not more than 50% of the total AUC is obtained over the first 30 days after injection.

For the object of the present invention, the following terms are used indistinctly: "PLA"; "biodegradable thermoplastic polymer of polylactic acid"; "lactic polyacid" and "polylactic acid".

"DMSO" and "dimethyl sulphoxide".

The invention is defined in the claims.

Consequently, a first aspect of the invention is a composition suitable for forming an intramuscular implant comprising a biodegradable thermoplastic polymer of polylactic acid (PLA), DMSO and an aromatase inhibitor compound of general formula (1): 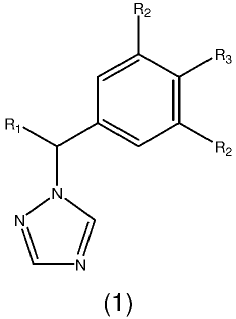 wherein:

When R₁ is H; R₂ is 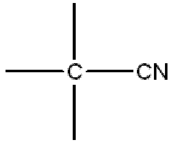 and R₃ is H

When R₁ is 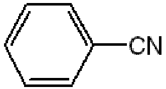 R₂ is H and R₃ is CN characterised in that the aromatase inhibitor compound is in suspension in a solution containing DMSO and PLA and represents between 15-50% by weight of the total composition, with the composition being able to solidify, to form a solid or gel-type implant on contact with an aqueous fluid or with that of the body, with therapeutic values after *in vivo* administration of at least 100 nmol/mL for more than 60 days.

The aromatase inhibitor compound is in suspension in a solution containing DMSO and PLA and represents between 20-30% by weight of the total composition.

More preferably, the aromatase inhibitor compound is in suspension in a solution containing DMSO and PLA and is 25% by weight of the total composition.

According to another aspect, the aromatase inhibitor compound of general formula (1) has the following particle size distribution:
- < 10% of the particles less than 20 microns,
- < 10% of the particles greater than 350 microns and
- d0.5 between 70-200 microns.

The aromatase inhibitor compound is letrozole or anastrozole, either alone or in combination.

The solution formed by DMSO and PLA has 40-43% by weight of PLA (100% lactic) + 57-60% by weight of DMSO.

In a further preferred embodiment, the terminal group of the PLA is as an ester instead of a carboxylic acid.

In another additional preferred embodiment, the active ingredient is subjected to sterilisation, for example by gamma or beta radiation. Sterilisation by radiation of the active ingredient may be carried out prior to its inclusion in the implantable composition to a maximum value of 35 kGy. Sterilisation of the active ingredient can also be carried out by terminal radiation of the product.

In a further preferred embodiment, the ratio of DMSO to letrozole is in the range of 0.5 to 3.7 and preferably in the range of 1.7 to 1.8.

In a still further preferred embodiment, the viscosity of the solution containing DMSO and PLA is in the range of between 0.8 and 1.8 Pa.s and preferably and preferably between 0.8 and 1.5 Pa.s, more preferably between 0.8 and 1.3 Pa.s and between 1.00 and 1.20 Pa.s.

In another additional embodiment, the maximum volume is 2 mL, allowing administration of 500 mg of letrozole by intramuscular injection.

A second aspect of the invention relates to the use of the composition above as a medicine for the treatment of breast cancer.

According to another aspect, the composition suitable for forming an intramuscular implant is characterised in that the AUC of the burst of the compound with the general formula (1) must be less than 10% of the total AUC and not more than 50% of the total AUC over the first 30 days after injection. The final formulation can be prepared, for example, included in a syringe ready for use for intramuscular injection. The same formulation may form part, for example, of a kit of two syringes, one male and one female or two male syringes linked by a connector in which the solution of polymer in DMSO is in one syringe and the aromatase inhibitor is in solid form in a second syringe. Similarly, the final composition can be obtained by, for example, maintaining one syringe with the polymer and aromatase inhibitor in solid state and the solvent in a second syringe.

Reconstitution in these cases is via direct joining of male and female syringes or by a connector where there are two male syringes, and the push-pull movement of the plungers in both directions giving rise to the combination of the products, and in this way to the solution of the polymer and the suspension of the active ingredient.

Any alternative to this system that gives rise to the composition of the present invention is possible, so that any variation with other designs of the formulation, where the final combination results in the desired product, for example maintaining the solvent or polymer solution in a vial apart from the active ingredient, or for example keeping the polymeric solution in a preloaded syringe and the aromatase inhibitor in a vial so that the polymeric solution is injected into the vial to give rise to the formation of the suspension, will be considered as possible alternatives for the object of the present invention.

Therefore, according to another aspect, the present invention refers to a suitable kit for the *in situ* preparation of the composition of the present invention, where the aromatase inhibitor compound of general formula (1) and the polymer are in a first container in solid form and the DMSO is in a second separate container.

Therefore, according to another aspect, the present invention refers to a suitable kit for the *in situ* preparation of the composition of the present invention where the polymer is lyophilised.

Therefore, according to another aspect, the present invention refers to a suitable kit for the *in situ* preparation of the composition of the present invention wherein the aromatase inhibitor compound of general formula (1) is in a first container in solid form and the DMSO and the polymer are in a second container in solution.

Therefore, according to another aspect, a suitable kit for the *in situ* preparation of the composition of the present invention wherein the aromatase inhibitor compound of general formula (1), the polymer and the DMSO are in a single container in the form of a suspension is disclosed.

Throughout the development of the present invention, the behaviour of various parameters that might have an influence on the result of the implantable compositions for prolonged release in accordance with the invention have been investigated. The parameters were the following:

### 1. Rheological properties of the polymeric solutions and inherent viscosity

The behaviour of the fluid, both of polymer solutions and the complete formulation, was evaluated by rheometry. In the case of matrices being considered as a vehicle for letrozole, these all have Newtonian behaviour. In the present document, viscosity is used as an indirect parameter and additional to the concentration of the polymer for the behaviour of the injectable formulation with respect to its ability to control the initial release of product.

Based on experimental work, it was determined that the polymer solution should have a minimum viscosity of 0.8 Pa.s, although this should preferably be around 1 Pa.s, but not greater than 1.8 Pa.s. Polymer solutions, in DMSO and measured at 25 ºC, which are obtained within this viscosity range, provide the appropriate balance between solubilisation of the active ingredient and its retention in the polymeric matrix, thereby obtaining clinically relevant plasma concentrations of letrozole or anastrozole, avoiding release of excessive amounts of the active ingredient that may compromise the useful life of the implant during the diffusion phase of the active ingredient. When the active ingredient is added, the viscosity increases, but must not exceed the range of 3-4 Pa.s.

The following tables show the apparent viscosity of the most suitable polymers in their *in vitro* and *in vivo* behaviour at different concentrations in DMSO at 25 ºC, as well as the viscosity of a final preferred formulation.

Viscosity of the PLA polymer Resomer^{®} R 203 S, irradiated as raw material by beta radiation at a dose of 10 kGy,

| Viscosity (Pa.s) | D,L | -lactic polymer (% by w) | | |
|---|---|---|---|---|
| | 40% | 41.5% | 43% | 45% |
| mean | 0.874 | 1.020 | 1.295 | 1.645 |
| SD | 0.043 | 0.021 | 0.038 | 0.016 |

Viscosity of a preferred formulation

| Viscosity (Pa.s) | | |
|---|---|---|
| **Replicated** | **mean** | **SD** |
| 2.462 | 2.514 | 0.045 |
| 2.536 | | |
| 2.543 | | |

### 2. Particle size of the active ingredient

The presence of the active ingredient in solution or in suspension critically affects the process of release of the active ingredient once the formulation is injected intramuscularly. Thus when letrozole is dissolved in the solution containing the polymer and DMSO, the intramuscular injection of the formulation gives rise to the release of an excessive amount over the first few days due to the diffusion of letrozole together with water-miscible solvent during the hardening process of the polymeric carrier, followed by a latency period in which *in vivo* release of the active ingredient is minimal and the final release of letrozole occurs at the time when the polymer experiences degradation by hydrolysis. The present document describes how only formulations containing letrozole in suspension are capable of controlling the initial release of the active ingredient and of preventing these periods of latency in which the formulation is not clinically effective. The particle size of the active ingredient therefore has a key effect in the final behaviour of the formulation as it directly affects the process of release from the formulation once it has been administered. The importance of this fundamental fact has not been previously described in documents describing the preparation of prolonged release formulations containing aromatase inhibitors.

Thus, the use of different size fractions was evaluated in order to determine or narrow an interval that is suitable and as well defined as possible. Better than a specific and totally narrow size, it is more useful to have a distribution of various crystal sizes enabling, to a certain extent, modulation of release in a staggered way. In this way, the smallest sizes are not useful because they very easily diffuse with the solvent during the formation of the implant (below 50 microns). Particles of intermediate sizes, with mean values close to 100-300 microns, can be more useful because they are retained by the matrix, require more time to dissolve and remain trapped in the implant during its solidification. Sizes above this require a high degree of degradation of the polymer, resulting in new latency periods in the life cycle of the implant and excessive releases at the time of hydrolysis of the polymer, the use of a high percentage in this size range not being particularly safe.

In a preferred embodiment, it has been determined that elimination not only of the smallest sizes but also of the intermediate to low sizes (50-100 microns) provides improvement to the product, given that, on the one hand, the final viscosity of the reconstituted product is reduced and, on the other hand, the pronounced burst effect is substantially prevented.

*3. Degree of suspension of the active ingredient in the solution containing the PLA and the DMSO.*

A characteristic of the present composition is that the aromatase inhibitor compound of general formula (1) is suspended, preferably present in an amount close to 25% by weight of the total composition, in a solution containing DMSO and PLA, which make up the remaining 75% by weight of the total composition.

This is not a random fact, but essential, because as will be seen later in example 1 if, for example, the formulation is in solution, the *in vivo* response is not satisfactory.

### EXAMPLES

The following examples are illustrative of the invention and are not to be considered limiting.

### Example 1: Study of the influence of the physical form of the active ingredient in the formulation: Suspension vs. Solution

The influence of the physical form of letrozole in the injectable solution (solution vs. suspension of the active ingredient) was evaluated by the use of the formulation described below:

**Formulation 1: formulation with letrozole in solution**

| | Ingredient | Amount (mg) |
|---|---|---|
| Syringe 2.25 ml male | Lactic acid polymer (ester terminal group) of intrinsic viscosity of 0.3 dl/g, irradiated as raw material at 10 kGy. | 55.20 |
| | Dimethyl sulphoxide | 82.80 |
| Syringe 2.25 ml female | Ingredient | Amount (mg) |
| | Letrozole | 16.20 |

**Formulation 2: formulation with letrozole in suspension**

| | Ingredient | Amount (mg) |
|---|---|---|
| Syringe 2.25 ml male | Lactic acid polymer (ester terminal group) of intrinsic viscosity of 0.3 dl/g, irradiated as raw material at 10 kGy. | 38.80 |
| | Dimethyl sulphoxide | 58.30 |
| Syringe 2.25 ml female | Ingredient | Amount (mg) |
| | Letrozole | 32.40 |

Letrozole particle size in formulation 2 was characterised by the technique of laser ray diffraction (Malvern Mastersizer 2000, suspended in water until obscuration of 9.41%) and had the following distribution (in % volume): d(0.1) = 38.21 µm, d(0.5) = 141.35 µm and d(0.9) = 312.13 µm.

In both cases, reconstitution of the product was carried out by connection of the male and female syringes and successive movements of the plungers in both directions until complete solution of the polymer had been achieved.

### Study of in vivo release in New Zealand white rabbits

Trials of *in vivo* release for the present document were carried out by determination of pharmacokinetic profiles of letrozole and anastrozole in plasma after intramuscular administration in experimental animal models, rabbits and/or dogs.

For quantification of letrozole in dog and rabbit plasma a technique based on high performance liquid chromatography with fluorescence detector HPLC-FLD with mass-mass detector using a liquid-liquid extraction with ethylene acetate as the organic solvent and resuspension for analysis in reverse phase with isocratic elution. Carvedilol was used as the internal standard and the process time was 8.5 min with a letrozole retention time of 7.8 min and applying wavelengths of 240 nm (λ excitation) and 315 nm (λ emission). The validated concentration interval was from 5 (LLOQ) to 500 ng/mL, that is with a lower limit of quantification or minimum quantifiable analyte concentration of the analytical technique of 5 ng/mL and a maximum validated concentration of 500 ng/mL. Calibration curves obtained were characterised with a correlation coefficient of over 0.99. Precision and intra-assay and inter-assay accuracy was less than 20% for the LLOQ and less than 15% for the other QCs, that is, of the other known concentration samples used as quality controls in conducting the analysis. The bioanalytical method was validated over three days and the results met the acceptance criteria described in the FDA guide: "Bioanalytical Method Validation".

Samples of letrozole in dog and rabbit plasma were demonstrated to be stable at room temperature for up to 6 hours. Processed samples could be stored in the sampler at 4 ºC for 24 hours without noticing any change in the precision and accuracy of the analysis. Cycles of freezing/thawing carried out did not affect the stability of letrozole in dog and rabbit plasma. Long term stability of the samples stored at -80 ºC demonstrated stability for 132 days.

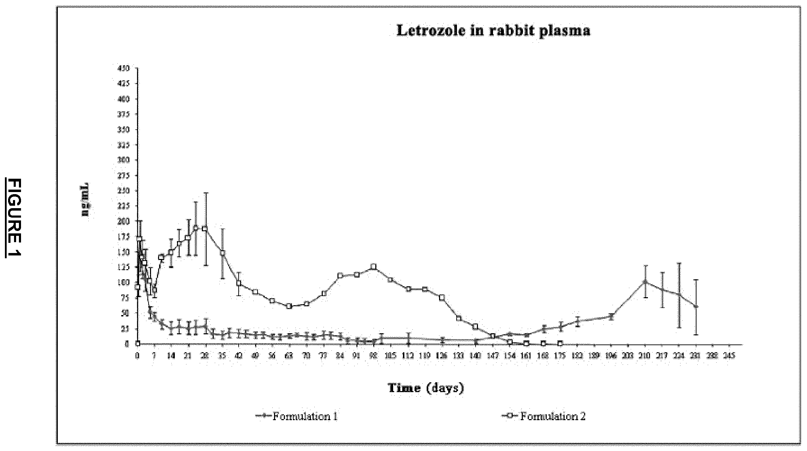

In the present example, formulations 1 and 2 were administered intramuscularly in the gluteus muscle of New Zealand white rabbits with approximate body weight of 3 kg. Three animals were used for each group and they were injected with an amount of formulation corresponding to 5.4 mg/kg for formulation 1 and 10.8 mg/kg for formulation 2. After injection, samples of plasma were collected from the rabbits at previously established sampling times of up to 231 days after the injection. The results obtained are shown in Figure 1. Figure 1 shows that administration of the double dose of formulation 2 compared to formulation 1 in New Zealand white rabbits gave rise to similar initial plasma levels. Given that the dose used in formulation 1 was half that used in formulation 2, the clear importance of controlling the physical form of the active ingredient in the formulation could be seen. Letrozole, being in solution in formulation 1, diffuses with the solvent to a greater extent than in the case of letrozole in formulation 2. It can also be seen that in the case of formulation 1 the plasma levels fell swiftly, and did not start to recover until day 154 after injection, the time at which the active ingredient starts to be released due to the hydrolytic degradation of the polymer that retains it. Formulation 2 was able to maintain continuous significantly high levels of letrozole in rabbit plasma for more than 4 months.

### Example 2: Study of the influence of the terminal group of the polymer

Evaluation of the influence of the terminal carboxylic or ester (N-capped) group of the polymer was evaluated by the use of the formulations described below:

**Formulation 1: formulation of lactic acid polymer terminating in a carboxylic group**

| | Ingredient | Amount (mg) |
|---|---|---|
| Syringe 2.25 ml male | Lactic acid polymer (carboxylic terminal group) of intrinsic viscosity of 0.3 dl/g, irradiated as raw material at 10 kGy. | 38.80 |
| | Dimethyl sulphoxide | 58.30 |
| Syringe 2.25 ml female | Ingredient | Amount (mg) |
| | Letrozole | 32.40 |

**Formulation 2: formulation with lactic acid polymer terminating in an ester group**

| | Ingredient | Amount (mg) |
|---|---|---|
| Syringe 2.25 ml male | Lactic acid polymer (ester terminal group) of intrinsic viscosity of 0.3 dl/g, irradiated as raw material at 10 kGy. | 38.80 |
| | Dimethyl sulphoxide | 58.30 |
| Syringe 2.25 ml female | Ingredient | Amount (mg) |
| | Letrozole | 32.40 |

Letrozole particle sizes in formulations 1 and 2 were characterised by the technique of laser ray diffraction (Malvern Mastersizer 2000, suspended in water until obscuration of 9.41%) and had the following distribution (in % volume): d(0.1) = 38.21 µm, d(0.5) = 141.35 µm and d(0.9) = 312.13 µm.

In both cases, reconstitution of the product was carried out by connection of the male and female syringes and successive movements of the plungers in both directions until complete solution of the polymer had been achieved.

In parallel, the viscosity of the solution containing the components of the male syringe was evaluated, in amounts proportionally equivalent to those described (41.5% by weight of polymer with respect to the weight of solution) at 25 ºC by the use of a rotational viscometer (Haake). The viscosity result values obtained are shown in the following table:

| Polymer (beta irradiated at 10 kGy as raw material) | Viscosity at 25 ºC (Pa.s) |
|---|---|
| PLA-carboxylic terminal group | 1.221 |
| PLA-ester terminal group | 1.054 |

### Study of in vivo release in New Zealand white rabbits

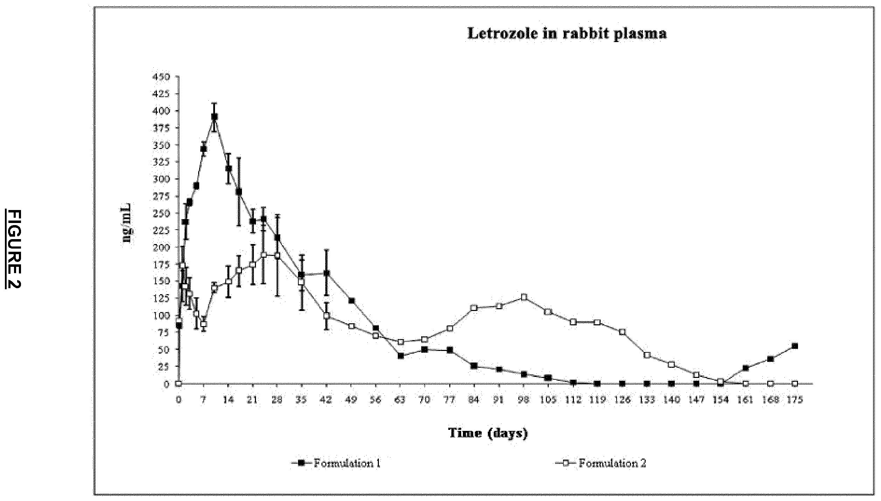

In the present example, formulations 1 and 2 were administered intramuscularly in the gluteus muscle of New Zealand white rabbits with approximate body weight of 3 kg. Three animals were used for each group and they were injected with an amount of formulation corresponding to 10.8 mg/kg for both formulations. After injection, samples of plasma were collected from the rabbits at previously established sampling times of up to 175 days after injection. The results obtained are shown in Figure 2.

Figure 2 shows that administration of formulation 1 gave rise to very high initial letrozole plasma levels after injection. However, the levels progressively reduced until the levels were undetectable at 119 days. Formulation 2, on the other hand, resulted in significantly high levels over a clearly longer period of time. The high hydrophilicity of the polymer with the carboxylic terminal group and its higher wettability compared to the polymer with ester termination is probably behind the higher release of the active ingredient over the early phases of the study. Formulation 2, however, was able to modulate release of letrozole to a higher degree than formulation 1, giving rise to levels that were more sustainable over time. The % area under the curve of letrozole plasma levels in each formulation over the total area under the curve are shown in the following table:

| Formulation | % AUC first 3 days | % AUC up to day 30 | AUC total (ng h/ml) |
|---|---|---|---|
| 1 | 3.981 | 66.630 | 328086.1 |
| 2 | 2.806 | 36.651 | 352267.6 |

### Example 3: Study of the influence of the terminal group of the polymer in Beagle dogs

Evaluation of the influence of the terminal carboxylic or ester (N-capped) group of the polymer was evaluated by the use of the formulations described below:

**Formulation 1: formulation of lactic acid polymer terminating in a carboxylic group**

| | Ingredient | Amount (mg) |
|---|---|---|
| Syringe 2.25 ml male | Lactic acid polymer (carboxylic terminal group) of intrinsic viscosity of 0.3 dl/g, irradiated as raw material at 10 kGy. | 107.6 |
| | Dimethyl sulphoxide | 151.7 |
| Syringe 2.25 ml female | Ingredient | Amount (mg) |
| | Letrozole | 86.5 |

**Formulation 2: formulation with lactic acid polymer terminating in an ester group**

| | Ingredient | Amount (mg) |
|---|---|---|
| Syringe 2.25 ml male | Lactic acid polymer (ester terminal group) of intrinsic viscosity of 0.3 dl/g, irradiated as raw material at 10 kGy. | 107.6 |
| | Dimethyl sulphoxide | 151.7 |
| Syringe 2.25 ml female | Ingredient | Amount (mg) |
| | Letrozole | 86.5 |

Letrozole particle sizes in formulations 1 and 2 were characterised by the technique of laser ray diffraction (Malvern Mastersizer 2000, suspended in water until obscuration of 9.41%) and had the following distribution (in % volume): d(0.1) = 38.21 µm, d(0.5) = 141.35 µm and d(0.9) = 312.13 µm.

In both cases, reconstitution of the product was carried out by connection of the male and female syringes and successive movements of the plungers in both directions until complete solution of the polymer had been achieved.

The apparent viscosity at 25 ºC of fully reconstituted formulation 2 gave a value of 2.865 Pa.s.

In parallel, the viscosity of the solution containing the components of the male syringe was evaluated, in amounts proportionally equivalent to those described (41.5% by weight of polymer with respect to the weight of solution) at 25 ºC by the use of a rotational viscometer (Haake). The viscosity result values obtained are shown in the following table:

| Polymer (beta irradiated at 10 kGy as raw material) | Viscosity at 25 ºC (Pa.s) |
|---|---|
| PLA-carboxylic terminal group | 1.221 |
| PLA-ester terminal group | 1.054 |

### Study of in vivo release in Beagle dogs

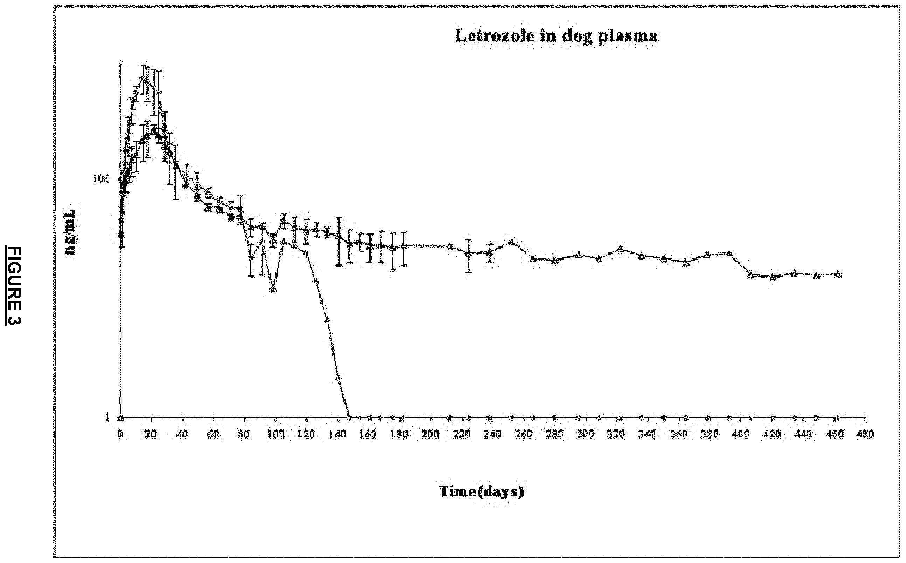

In the present example, formulations 1 and 2 were administered intramuscularly in the gluteus muscle of Beagle dogs with approximate body weight of 10 kg. Three animals were used for each group and they were injected with an amount of formulation corresponding to 86.5 mg/kg for both formulations. After injection, samples of plasma were collected from the dogs at previously established sampling times of up to 472 days after injection. The results obtained are shown in Figure 3.

Figure 3 shows that the difference observed in the previous example between the two polymers was significantly higher in dogs. The lower body temperature of the Beagle dog (some 2.4 ºC lower than the New Zealand white rabbit) slowed down the diffusion process, probably due to a combination of effects between the lower speed of hydrolysis of the polymers at the lower temperature and lower diffusion of the active ingredient across the matrix also due to the lower body temperature. A higher influence of polymer hydrophilicity in the kinetic profile of letrozole was observed in this situation of reduced diffusion and speed of degradation. The observation of a continuous increase in letrozole plasma levels over the first 14 to 21 days demonstrates an enterohepatic circulation phenomenon that has been demonstrated by population pharmacokinetic analysis of the data obtained (Nonmen). This enterohepatic circulation of letrozole is likely to be also observable in humans. The % area under the curve of letrozole plasma levels in each formulation over the total area under the curve are shown in the following table:

| Formulation | % AUC first 3 days | % AUC up to day 30 | AUC total (ng h/ml) |
|---|---|---|---|
| 1 | 1.527 | 74.963 | 460177.6 |
| 2 | 0.537 | 36.940 | 482879.8 |

## Claims

1. Composition suitable for forming an *in situ* intramuscular implant comprising a biodegradable thermoplastic polymer of polylactic acid (PLA), DMSO and an aromatase inhibitor compound that is letrozole or anastrozole, either alone or in combination, **characterised in that** the aromatase inhibitor compound is in suspension in a solution containing DMSO and PLA and represents between 20-30% by weight of the total composition, with the composition being able to solidify, to form a solid or gel-type *in situ* implant on contact with an aqueous fluid or that of the body, with therapeutic values after *in vivo* administration of at least 100 nmol/mL for more than 60 days, wherein the solution containing DMSO and PLA is 40-43% by weight of 100% lactic PLA and 57-60% by weight of DMSO.

2. Composition suitable for forming the *in situ* intramuscular implant of claim 1 **wherein** the aromatase inhibitor compound is in suspension in a solution containing DMSO and PLA and represents 25% by weight of the total composition.

3. Composition suitable for forming the *in situ* intramuscular implant of claim 1 **wherein** the aromatase inhibitor compound has the following particle size distribution in the formulation as measured by the technique of laser ray diffraction in water suspension until obscuration of 9.41%:
• < 10% of the particles less than 20 microns,
• < 10% of the particles greater than 350 microns and
• d0.5 between 70-200 microns.

4. Composition suitable for forming the *in situ* intramuscular implant of claim 1 wherein the terminating group of the PLA is an ester.

5. Composition suitable for forming the *in situ* intramuscular implant of any of the previous claims **wherein** the formulation is irradiated at a maximum value of 35 kGy.

6. Composition suitable for forming the *in situ* intramuscular implant of claim 1 **wherein** the ratio between DMSO and the aromatase inhibitor compound is in the range of 0.5 to 3.7.

7. Composition suitable for forming the *in situ* intramuscular implant of claim 1 **wherein** the ratio between DMSO and the aromatase inhibitor compound is in the range of 1.7 to 1.8.

8. Composition suitable for forming the *in situ* intramuscular implant of claim 1 **wherein** the viscosity of the solution containing DMSO and PLA is in the range between 0.8 and 1.8 Pa.s measured at 25 ºC using a rotational viscometer.

9. Composition of claims 1 to 8 for use as a medicine in the treatment of breast cancer.

10. Kit suitable for the *in situ* preparation of the composition of any of the claims 1 to 8 wherein the aromatase inhibitor compound and the polymer in solid form are in a first container and the DMSO is in a second separate container.

11. Kit of claim 10 wherein the polymer is lyophilised.

12. Kit suitable for the *in situ* preparation of the composition of any of the claims 1 to 8 wherein the aromatase inhibitor compound is in a first container in solid form and the DMSO and the polymer are in a second container in solution.

## Patentansprüche

1. Zusammensetzung, die zur Bildung eines intramuskulären *In*-*situ*-Implantats geeignet ist, umfassend ein biologisch abbaubares thermoplastisches Polymer aus Polymilchsäure (PLA), DMSO und einer Aromatase-Inhibitorverbindung, die Letrozol oder Anastrozol ist, entweder allein oder in Kombination, **dadurch gekennzeichnet, dass** die Aromatase-Inhibitorverbindung in einer Lösung, die DMSO und PLA enthält, suspendiert ist und zwischen 20 und 30 Gew.- % der Gesamtzusammensetzung ausmacht, wobei die Zusammensetzung in der Lage ist, sich zu verfestigen, um bei Kontakt mit einer wässrigen Flüssigkeit oder der des Körpers ein festes oder gelartiges *In-situ-Implantat* mit therapeutischen Werten nach *In-vivo-*Gabe von mindestens 100 nmol/ml für mehr als 60 Tage zu bilden, wobei die Lösung, die DMSO und PLA enthält, 40 bis 43 Gew.- % 100%ige milchige PLA und 57 bis 60 Gew.- % DMSO beträgt.

2. Zusammensetzung, die zur Bildung des intramuskulären *In*-*situ*-Implantats nach Anspruch 1 geeignet ist, **wobei** die Aromatase-Inhibitorverbindung in einer Lösung, die DMSO und PLA enthält, suspendiert ist und 25 Gew.- % der Gesamtzusammensetzung ausmacht.

3. Zusammensetzung, die zur Bildung des intramuskulären *In*-*situ*-Implantats nach Anspruch 1 geeignet ist, **wobei** die Aromatase-Inhibitorverbindung die folgende Teilchengrößenverteilung in der Formulierung, gemessen durch die Technik der Laserstrahlbeugung in der Wassersuspension, bis zur Schleierbildung von 9,41 % aufweist:
• < 10 % der Partikel weniger als 20 Mikrometer,
• < 10 % der Partikel größer als 350 Mikrometer und
• d0,5 zwischen 70-200 Mikrometer.

4. Zusammensetzung, die zur Bildung des intramuskulären *In*-*situ*-Implantats nach Anspruch 1 geeignet ist, wobei die Endgruppe des PLA ein Ester ist.

5. Zusammensetzung, die zur Bildung des intramuskulären *In*-*situ*-Implantats nach einem der vorhergehenden Ansprüche geeignet ist, **wobei** die Formulierung bei einem Maximalwert von 35 kGy bestrahlt wird.

6. Zusammensetzung, die zur Bildung des intramuskulären *In*-*situ*-Implantats nach Anspruch 1 geeignet ist, **wobei** das Verhältnis zwischen DMSO und der Aromatase-Inhibitorverbindung im Bereich von 0,5 bis 3,7 liegt.

7. Zusammensetzung, die zur Bildung des intramuskulären In-situ-Implantats nach Anspruch 1 geeignet ist, **wobei** das Verhältnis zwischen DMSO und der Aromatase-Inhibitorverbindung im Bereich von 1,7 bis 1,8 liegt.

8. Zusammensetzung, die zur Bildung des intramuskulären *In*-*situ*-Implantats nach Anspruch 1 geeignet ist, **wobei** die Viskosität der Lösung, die DMSO und PLA enthält, im Bereich zwischen 0,8 und 1,8 Pa.s liegt, gemessen bei 25 °C unter Verwendung eines Rotationsviskosimeters.

9. Zusammensetzung nach den Ansprüchen 1 bis 8 zur Verwendung als Arzneimittel bei der Behandlung von Brustkrebs.

10. Kit, das für die In-situ-Herstellung der Zusammensetzung nach einem der Ansprüche 1 bis 8 geeignet ist, wobei die Aromatase-Inhibitorverbindung und das Polymer in fester Form in einem ersten Behälter vorliegen und das DMSO in einem zweiten separaten Behälter vorliegt.

11. Kit nach Anspruch 10, wobei das Polymer lyophilisiert ist.

12. Kit, geeignet für die In-situ-Herstellung der Zusammensetzung nach einem der Ansprüche 1 bis 8, wobei die Aromatase-Inhibitorverbindung in einem ersten Behälter in fester Form vorliegt und das DMSO und das Polymer in einem zweiten Behälter in Lösung vorliegen.

## Revendications

1. Composition conçue pour former un implant intramusculaire *in situ* comprenant un polymère thermoplastique biodégradable d'acide polylactique (PLA), du DMSO et un composé inhibiteur d'aromatase qui est le létrozole ou l'anastrozole, seul ou en combinaison, **caractérisée en ce que** le composé inhibiteur d'aromatase est en suspension dans une solution contenant du DMSO et du PLA et représente entre 20 et 30% en poids de la composition totale, la composition étant capable de se solidifier, pour former un implant *in situ* de type solide ou gel au contact d'un fluide aqueux ou de celui du corps, avec des valeurs thérapeutiques après administration *in vivo* d'au moins 100 nmol/ml pendant plus de 60 jours, dans laquelle la solution contenant du DMSO et du PLA comprend de 40 à 43 % en poids de PLA lactique à 100 % et de 57 à 60 % en poids de DMSO.

2. Composition conçue pour former l'implant intramusculaire *in situ* selon la revendication 1, **dans laquelle** le composé inhibiteur d'aromatase est en suspension dans une solution contenant du DMSO et du PLA et représente 25 % en poids de la composition totale.

3. Composition conçue pour former l'implant intramusculaire *in situ* selon la revendication 1, **dans laquelle** le composé inhibiteur d'aromatase a la distribution granulométrique suivante dans la formulation telle que mesurée par la technique de diffraction de rayon laser dans une suspension aqueuse jusqu'à un obscurcissement de 9,41 % :
• < 10 % des particules inférieures à 20 microns,
• < 10 % des particules supérieures à 350 microns et
• d0,5 entre 70 et 200 microns.

4. Composition conçue pour former l'implant intramusculaire *in situ* selon la revendication 1, **dans laquelle** le groupe de terminaison du PLA est un ester.

5. Composition conçue pour former l'implant intramusculaire *in situ* selon l'une quelconque des revendications précédentes, **dans laquelle** la formulation est irradiée à une valeur maximale de 35 kGy.

6. Composition conçue pour former l'implant intramusculaire *in situ* selon la revendication 1, **dans laquelle** le rapport entre le DMSO et le composé inhibiteur d'aromatase est compris entre 0,5 et 3,7.

7. Composition conçue pour former l'implant intramusculaire *in situ* selon la revendication 1, **dans laquelle** le rapport entre le DMSO et le composé inhibiteur d'aromatase est compris entre 1,7 et 1,8.

8. Composition conçue pour former l'implant intramusculaire *in situ* selon la revendication 1, **dans laquelle** la viscosité de la solution contenant du DMSO et du PLA est comprise entre 0,8 et 1,8 Pa.s, mesurée à 25 ^{S}C à l'aide d'un viscosimètre rotatif.

9. Composition selon les revendications 1 à 8, destinée à être utilisée comme médicament dans le traitement du cancer du sein.

10. Kit conçu pour la préparation *in situ* de la composition selon l'une quelconque des revendications 1 à 8, dans lequel le composé inhibiteur d'aromatase et le polymère sous forme solide sont dans un premier récipient et le DMSO est dans un second récipient séparé.

11. Kit selon la revendication 10, dans lequel le polymère est lyophilisé.

12. Kit conçu pour la préparation *in situ* de la composition selon l'une quelconque des revendications 1 à 8, dans lequel le composé inhibiteur d'aromatase est dans un premier récipient sous forme solide et le DMSO et le polymère sont dans un second récipient en solution.
